# EUROPÄISCHE PATENTSCHRIFT

(11) **EP 1 711 142 B1**
(45) Veröffentlichungstag und Bekanntmachung des Hinweises auf die Patenterteilung: **05.12.2007**
(21) Anmeldenummer: 05714916.3
(22) Anmeldetag: 01.02.2005
(51) Int. Cl.: A61F 5/00

(54) **Schutzkragen zum therapeutischen Kopf- bzw. Gesichtsschutz bei Kleinstkinderen**
Protective collar for therapeutic protection of head or face of little children
Collerette pour protection thérapeutique de la tête ou du visage pour petits enfants

(30) Priorität: 03.02.2004 DE 102004005352
(43) Veröffentlichungstag der Anmeldung: 18.10.2006
(73) Patentinhaber: Kühn, Julia, 73235 Weilheim/Teck (DE)
(72) Erfinder: Kühn, Julia, 73235 Weilheim/Teck (DE)
(74) Vertreter: Jakelski, Joachim
(86) Internationale Anmeldenummer: PCT/DE2005/000154
(87) Internationale Veröffentlichungsnummer: WO 2005/074842

(56) Entgegenhaltungen:
- EP-A- 0 468 697
- DE-A- 19 629 581
- DE-C- 461 057
- US-A- 5 500 954
- US-A- 5 644 793
- US-A1- 2002 053 348
- US-A1- 2003 150 401

## Beschreibung

Die Erfindung betrifft einen zum Kopf- oder Gesichtsschutz bei Kleinstkindern geeigneten Schutzkragen mit einem im Wesentlichen flachen, einseitig geöffneten und damit zwei Ringenden bildenden Ringteil, welches im Bereich der Ringenden Befestigungsmittel aufweist, mittels derer die Ringenden unter Ausbildung eines kegelstumpfförmigen Kragens aneinander fixierbar sind.

Therapeutische Schutzkragen der hier betroffenen Art dienen dazu, bei Kleinstkindern, nämlich bei Säuglingen bis zum Alter von etwa einem Lebensjahr, welche im Bereich des Kopfes bzw. des Gesichtes eine äußerliche Verletzung oder eine operative Wunde bspw. aufgrund eines operativen Eingriffs zur Behebung einer Gaumenspaltenanomalie aufweisen, deren Armbewegungsfreiheit so einzuschränken, daß verhindert wird, daß diese Kinder gemäß ihrer natürlichen Verhaltensweise die entsprechende Kopfstelle mit den Händen berühren, einen an dieser Kopfstelle etwa angebrachten Verband abreißen oder gar die Operationsstelle berühren. Dadurch wird der natürliche Heilungsprozeß wesentlich beeinträchtigt oder es treten im schlimmsten Fall sogar unerwünschte Infektionen und damit einhergehende erhebliche Komplikationen beim Heilungsprozeß auf.

Ein Schutzkragen zum Kopf- und Gesichtsschutz für ein Tier geht beispielsweise aus der EP-A-468697 hervor. Dieser Schutzkragen weist ein im Wesentlichen flaches, einseitig geöffnetes und damit zwei Ringenden bildendes Ringteil auf, bei welchem im Bereich der Ringenden Befestigungsmittel vorgesehen sind, mittels derer die Ringenden unter Ausbildung eines kegelstumpfförmigen Kragens aneinander fixierbar sind. Das Ringteil selbst besteht zwar aus einem flexiblen, aber dennoch genügend stabilen, unverwüstlichen und insbesondere eine recht hohe Eigensteifigkeit aufweisenden Material, um zu verhindern, dass beispielsweise ein Hund oder eine Katze ein zu schützendes Körperteil zerbeißt oder beleckt. Darüber hinaus wird durch einen solchen steifen Schutzkragen auch wirkungsvoll verhindert, dass beispielsweise ein Hund mit einer Hinterpfote an einer am Kopf angeordneten Wunde kratzt.

Ein Schutzkragen, der allerdings ebenfalls ausschließlich für Tiere geeignet ist, geht darüber hinaus aus der DE-A-196 29 581 hervor. Dieser soll sich insbesondere dadurch auszeichnen, daß das gesamte Wohlbefinden des Kleintieres aufgrund einer in Längsrichtung des Tieres besonderen Ausgestaltung des kegelstumpfartigen Kragens mit zwei in dieser Richtung unlösbar verbundenen Teilen jeweils unterschiedlicher Steifheit nur wenig beeinträchtigt wird. Dadurch soll sich dieser Kragen den Bewegungsabläufen des ihn tragenden Tieres flexibel anpassen, wodurch ein jeweils zu schützendes Körperteil davor geschützt werden soll, zerbissen oder auch nur beleckt zu werden. Ist nun eine stärkere Einschränkung der Bewegungsfreiheit des Kopfes notwendig, so schließt sich der flexiblere Teil des Schutzkragens an den steiferen Teil an. Soll indessen die Bewegungsfreiheit nur wenig eingeschränkt werden, so kann der flexible Teil über den steifen Teil gezogen werden, so daß das Tier ein vergrößertes Gesichtsfeld erlangt. Ein solcher Schutzkragen ist bei Menschen nicht einsetzbar.

Einen weiteren Weg zum Gesichtsschutz insbesondere bei Personen stellt die Verwendung von Gesichtsmasken dar, wie die bspw. in den vorveröffentlichten Druckschriften DE-A-100 02 350, US 5500954 und US-A-2002/0053348 offenbarten Masken.

Bei den genannten operativen bzw. therapeutischen an Kleinstkindern durchzuführenden ärztlichen Maßnahmen ist weiterhin bekannt, zur Vermeidung von Gesichtsberührungen durch die Hände bzw. Finger des Kindes dessen Arme durch perforierte Kunststoffröhren so zu fixieren, daß zwar die Arme bewegt, nicht jedoch gebeugt werden können.

Eine andere Methode der Fixierung der Ärmchen des Kleinstkindes, die bisweilen praktiziert wird, ist das Fixieren der Arme mittels Mullbinden.

Beide Methoden zur Fixierung der Arme des Kleinstkindes weisen den Nachteil auf, daß die Bewegungsfreiheit der Arme des Kindes erheblich eingeschränkt wird und damit die freie motorische Entwicklung des Kleinstkindes erheblich gestört wird oder sich sogar psychische Störungen einstellen.

Der vorliegenden Erfindung liegt deshalb die Aufgabe zugrunde, einen für Kleinstkinder zu dem genannten therapeutischen Zweck geeigneten Gesichtsschutz bereitzustellen, welcher möglichst wirksam Berührungen des Gesichtes oder Kopfes durch die Hände bzw. Finger des Kindes verhindert, gleichzeitig jedoch die Armbewegungsfreiheit möglichst wenig beeinträchtigt und einen hohen Tragekomfort aufweist, sodass beispielsweise auch ein Liegen des Kindes durch den Schutzkragen in keiner Weise eingeschränkt wird und so ohne Weiteres möglich ist.

Diese Aufgabe wird zum einen durch einen Schutzkragen der Eingangs beschriebenen Art dadurch gelöst, dass das Ringteil aus einem mit einem flachen Formkörper gefüllten flachen Kissen gebildet ist. Ein solcher Schutzkragen übt aufgrund der im zusammengebauten Zustand formbedingten Eigensteifigkeit nur äußerst geringe Zugkräfte am Hals des Kleinstkindes aus. Dadurch, dass das Ringteil aus einem mit einem flachen Formkörper gefüllten flachen Kissen gebildet ist, weist der vorgeschlagene Kragen insbesondere keine harten oder scharfkantigen Bereiche oder Teile auf, an denen sich das Kleinstkind etwa verletzen könnte.

Die Aufgabe wird auch dadurch gelöst, dass bei einem gattungsgemäßen Schutzkragen das Ringteil aus einem im Wesentlichen formstabilen Innenteil und einem darüber lösbar anzuordnenden Hüllenmaterial gebildet ist. Auch durch einen solchen Schutzkragen werden Berührungen des Gesichtes oder Kopfes durch die Hände bzw. Finger des Kindes wirksam verhindert und gleichzeitig die Armbewegungsfreiheit und ganz allgemein die Bewegungsfreiheit des Kindes wenig beeinträchtigt.

Durch das lösbar angeordnete Hüllenmaterial lassen sich darüber hinaus die relativ hohen Anforderungen an die Hygiene, insbesondere im Krankenhausumfeld, problemlos erfüllen.

Aufgrund seiner Bauform ist der Schutzkragen einfach und schnell um den Hals des Kleinstkindes herum anbringbar. Über den bevorzugt flächigen Klettverschluß oder dgl. weist der Schutzkragen in der zusammengebauten Form eine genügende Stabilität, allerdings auch nicht zu hohe Eigensteifigkeit auf, um das Wohlbefinden des Kindes möglichst wenig zu beeinträchtigen.

Der erfindungsgemäße Schutzkragen ist zudem leicht an unterschiedliche Halsdurchmesser anpaßbar, indem die erforderliche Halsöffnung beim Zusammenbau des Kragens um den Hals des Kleinstkindes herum entsprechend gewählt wird.

Die Erfindung wird nachfolgend, unter Bezugnahme auf die beigefügte Zeichnung, anhand eines bevorzugten Ausführungsbeispiels, aus dem sich weitere Merkmale und Vorteile der Erfindung ergeben, noch eingehender beschrieben.

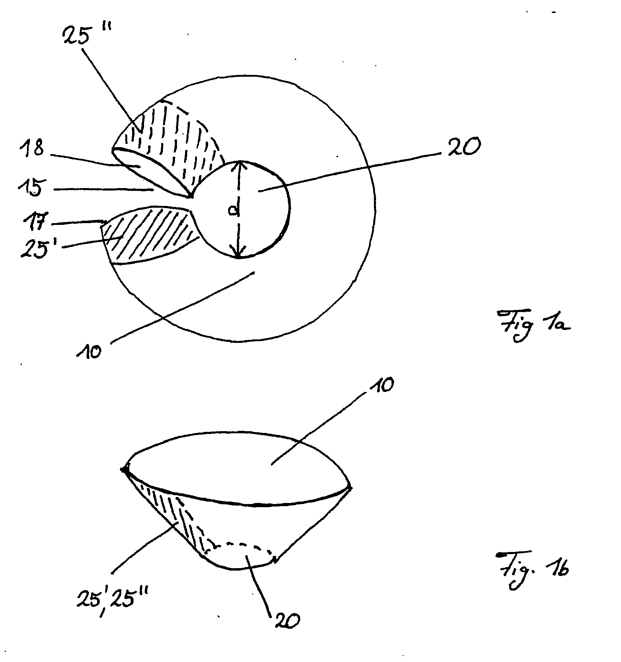

Im Einzelnen zeigen
- Fig. 1a: eine Draufsicht auf einen erfindungsgemäßen Schutzkragen vor dem Zusammenbau;
- Fig. 1 b: eine perspektivische seitliche Ansicht eines erfindungsgemäßen Schutzkragens in zusammengebauter Form; und
- Fig. 2: eine ebenfalls seitlich-perspektivische Ansicht des Anbauzustandes des erfindungsgemäßen Kragens am Hals eines schematisch gezeichneten Kleinstkindes.

Der in der Fig. 1a in der Draufsicht dargestellte erfindungsgemäße Schutzkragen besteht im nicht zusammengebauten Zustand aus einem einseitig unterbrochenen bzw. geöffneten 15 Ringteil 10. Das im wesentlichen flach ausgebildete Ringteil 10 besteht in dem Ausführungsbeispiel aus Filzvlies. Alternativ kann der Kragen aus einem mit einem leicht formstabilen flachen Inlay oder einem Füllmaterial gefüllten flachen Kissen oder dgl. gebildet sein.

Das Ringteil 10 weist eine kreisförmige innere Öffnung 20 mit einem Durchmesser d auf, welche im nach dem Anbau am Kind im wesentlichen durch dessen Hals ausgefüllt wird, wobei sich der Durchmesser d des Ringteils 10 beim Zusammenbau selbstverständlich etwas verringert.

Der einseitige Öffnungsschlitz 15 des Ringteils 10 dient dazu, das Anlegen des Kragens 10 um den Hals des Kleinstkindes herum zu ermöglichen. Zu diesem Zweck weist das Ringteil 10 im Bereich der Ringöffnung 15, und zwar in dem bevorzugten Ausführungsbeispiel an den beiden Ringenden 17, 18, einen üblicherweise zweiteiligen Klettverschluss 25', 25" auf, wobei das Bezugszeichen 25' das Klettvlies und das Bezugszeichen 25" das zugeordnete Haftvlies kennzeichnen. Es versteht sich, daß dieses Befestigungsmittel 25', 25" nicht notwendig an den Ringenden 17, 18 selbst angeordnet sein muß, sondern durchaus auch einen gewissen Abstand von den Ringenden 17, 18 aufweisen kann.

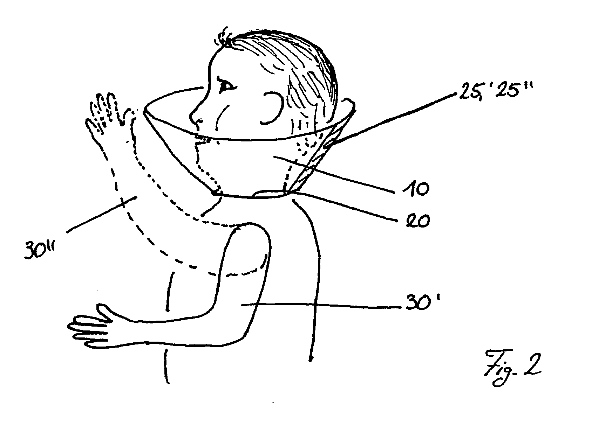

Mittels des Klettverschlusses 25', 25" kann das Ringteil 10 in der in Fig. 1b gezeigten Form um den Hals eines in der Fig. 2 schematisch angedeuteten Kleinstkindes herum zusammengeführt und danach zusammengebaut werden, wie in der Fig. 2 verdeutlicht ist.

Aus der Fig. 1 b ist ferner zu ersehen, wie der Schutzkragen 10 an den beiden Teilen 25', 25" des Klettverschlusses zusammengehalten wird. Zudem ist die sich beim Zusammenbau ergebende innere, im wesentlichen kreisförmige Öffnung 20 nur gestrichelt eingezeichnet, da sie in dieser Darstellung durch die vordere Außenwand des Ringteils 10 verdeckt wird.

In einer hier nicht gezeigten weiteren Ausführungsform besteht das Ringteil 10 aus einem Innenteil mit einer Eigensteifheit und einer darüber anzuordnenden abnehmbaren Stoffhülle, Kunststoffhülle oder dgl., wobei die Hülle aus einem leicht reinigbaren bzw. waschbaren Material gebildet ist. Durch diesen zweiteiligen Aufbau des Ringteils 10 lassen sich die relativ hohen Anforderungen an die Hygiene insbesondere im Krankenhausumfeld problemlos erfüllen.

Der Schutzkragen ist verformbar, so daß beispielsweise dann, wenn das Kleinstkind beim Schlafen auf der Seite liegt, keine störende und unnatürliche Haltung des Kopfes durch den Schutzkragen hervorgerufen wird. Der Schutzkragen weist dabei eine Elastizität derart auf, daß das Kind beim Schlafen einerseits nicht oder nur sehr wenig beeinträchtigt wird, andererseits auch in einer solchen Schlafposition eine Berührung des Gesichts nicht möglich ist.

Zudem kann vorgesehen sein, daß im (später dem Kindeshals zugewandten) Innenbereich des Ringteils 10 (in der Figur ebenfalls nicht gezeigte) Befestigungsmittel zur Befestigung oder Fixierung einer bevorzugt schlauchförmigen therapeutischen Einrichtung, bspw. einer Sondeneinrichtung (Magensonde, etc.) oder eines Infusionsschlauchs angeordnet sind. Dadurch wird ermöglicht, daß das Kleinstkind auch diese Einrichtung mit seinen Händen bzw. Fingern überhaupt nicht oder nur sehr schlecht erreichen kann und demzufolge nicht bewegen, verschieben oder sogar ab- bzw. herausreißen kann.

In der Fig. 2 ist ferner verdeutlicht, wie sich der Schutzkragen im zusammengebauten Zustand an den Hals des Kleinstkindes anschmiegt. Dadurch ist gewährleistet, daß der Kragen nicht nach unten hin verrutschen bzw. Wegkippen kann und in allen Positionen des Kindes, d.h. sowohl im Liegen als auch im Stehen, somit wirksam verhindert wird, daß ein hier gestrichelt eingezeichneter Arm 30' des Kindes bei einer Aufwärtsbewegung 30" auch im Falle des schematisch gezeigten Abwinkelns des Unterarms nicht das Gesicht des Kindes erreichen kann.

Die genannte Rutschfestigkeit des Kragens wird letztlich dadurch noch verstärkt, daß der Schutzkragen aufgrund seiner kegelstumpfförmigen Gestalt, von der Kopfform (Kinn etc.) des Kindes begünstigt, in Halsrichtung im wesentlichen in der Anbauposition fixiert ist.

## Patentansprüche

1. Schutzkragen zum Kopf- oder Gesichtsschutz bei Kleinstkindern mit einem im wesentlichen flachen, einseitig geöffneten (15) und damit zwei Ringenden (17, 18) bildenden Ringteil (10), welches im Bereich der Ringenden (17, 18) Befestigungsmittel (25', 25") aufweist, mittels derer die Ringenden (17, 18) unter Ausbildung eines kegelstumpfförmigen Kragens aneinander fixierbar sind, **dadurch gekennzeichnet, daß** das Ringteil aus einem mit einem flachen Formkörper gefüllten flachen Kissen gebildet ist.

2. Schutzkragen zum Kopf- oder Gesichtsschutz bei Kleinstkindern mit einem im wesentlichen flachen, einseitig geöffnete (15) und damit zwei Ringenden (17, 18) bildenden Ringteil (10), welches im Bereich der Ringenden (17, 18) Befestigungsmittel (25', 25") aufweist, mittels derer die Ringenden (17, 18) unter Ausbildung eines kegelstumpfförmigen Kragens aneinander fixierbar sind, **dadurch gekennzeichnet, daß** das Ringteil aus einem im wesentlichen formstabilen Innenteil und einem darüber lösbar anzuordnenden Hüllenmaterial gebildet ist.

3. Schutzkragen nach Anspruch 1 oder 2, **dadurch gekennzeichnet, daß** das Ringteil einstückig ausgebildet ist.

4. Schutzkragen nach einem der Ansprüche 1 bis 3, **dadurch gekennzeichnet, daß** die Befestigungsmittel durch einen Klettverschluss gebildet werden.

5. Schutzkragen nach einem der vorhergehenden Ansprüche, **dadurch gekennzeichnet, daß** das Ringteil aus einem leichtgewichtigen Material, bevorzugt aus Filzvlies, gebildet ist.

6. Schutzkragen nach einem der vorhergehenden Ansprüche, **dadurch gekennzeichnet, daß** an dem Ringteil Befestigungsmittel zur Befestigung oder Fixierung einer im wesentlichen schlauchförmigen therapeutischen Einrichtung angeordnet sind.

## Claims

1. Protective collar for therapeutic protection of head or face of little children comprising a substantially flat annular part (10), which is open to one side (15) thereby forming two annular ends (17, 18) and which comprises, in the area of said annular ends (17, 18), fastening means (25', 25") by means of which the annular ends (17, 18) can be fixed one to the other thereby forming a collar in the shape of a truncated cone, **characterized in that** the annular part is formed from a flat cushion filled with a flat molded body.

2. Protective collar for therapeutic protection of head or face of little children comprising a substantially flat annular part (10), which is open to one side (15) thereby forming two annular ends (17, 18) and which comprises, in the area of said annular ends (17, 18), fastening means (25', 25") by means of which the annular ends (17, 18) can be fixed one to the other thereby forming a collar in the shape of a truncated cone, **characterized in that** the annular part consists of an inner part of substantially stable shape and an envelope material intended to be detachably placed on top thereof.

3. The protective collar as defined in Claim 1 or Claim 2, **characterized in that** the annular part has a one-piece design.

4. The protective collar as defined in any of Claims 1 to 3, **characterized in that** the fastening means is configured as a Velcro fastener.

5. The protective collar as defined in any of the preceding claims, **characterized in that** the annular part is made from a light-weight material, preferably from a non-woven felt.

6. The protective collar as defined in any of the preceding claims, **characterized in that** fastening means are provided on the annular part for fastening or fixing a substantially tube-shaped therapeutic device.

## Revendications

1. Collerette pour protection de la tête et du visage pour petits enfants comportant une partie annulaire (10) pour l'essentiel plate, à ouverture unilatérale (15) et formant donc deux extrémités d'anneau (17, 18), avec dans la zone des extrémités d'anneau (17, 18) des moyens de fixation ('25', 25") pour fixer les extrémités d'anneau (17, 18) l'une contre l'autre en formant une collerette en forme de tronc de cône,
**caractérisée en ce que**
la partie annulaire est formée par un coussin plat rempli d'un corps moulé plat.

2. Collerette pour protection de la tête et du visage pour petits enfants comportant une partie annulaire (10) pour l'essentiel plate, à ouverture unilatérale (15) et formant donc deux extrémités d'anneau (17, 18), avec dans la zone des extrémités d'anneau (17, 18) des moyens de fixation ('25', 25") pour fixer les extrémités d'anneau (17, 18) l'une contre l'autre en formant une collerette en forme de tronc de cône,
**caractérisée en ce que**
la partie annulaire est constituée d'une partie intérieure pour l'essentiel de forme stable et d'un matériau d'enveloppe à disposer de façon amovible sur celle-ci.

3. Collerette pour protection selon la revendication 1 ou 2,
**caractérisée en ce que**
la partie annulaire est formée d'une seule pièce.

4. Collerette pour protection selon l'une des revendications 1 à 3,
**caractérisée en ce que**
les moyens de fixation sont formés par une fermeture autogrippante.

5. Collerette pour protection selon l'une des revendications précédentes,
**caractérisée en ce que**
la partie annulaire est formée d'un matériau léger, de préférence de feutre.

6. Collerette pour protection selon l'une des revendications précédentes,
**caractérisée en ce que**
des moyens de fixation pour attacher ou fixer un dispositif thérapeutique pour l'essentiel en forme de tube flexible sont disposés sur la partie annulaire.
